Europäisches Patentamt

European Patent Office

Office européen des brevets

(11) Publication number: **0 206 558**
**A1**

## (12) EUROPEAN PATENT APPLICATION

(21) Application number: 86304109.1

(51) Int. Cl.⁴: **A 61 M 25/02**, A 61 M 5/32

(22) Date of filing: 30.05.86

(30) Priority: 30.05.85 US 739239

(43) Date of publication of application: **30.12.86**
**Bulletin 86/52**

(84) Designated Contracting States: **AT BE CH DE FR GB IT LI LU NL SE**

(71) Applicant: **Muller, Louis Frederick, 911 Main Street, El Segundo California 90245 (US)**

(72) Inventor: **Muller, Louis Frederick, 911 Main Street, El Segundo California 90245 (US)**

(74) Representative: **Hartley, David et al, c/o Withers & Rogers 4 Dyer's Buildings Holborn, London, EC1N 2JT (GB)**

(54) Retainer for medical devices.

(57) ·A disposable holder or retainer (10) is disclosed herein for retaining conventional medical equipment or assemblages (12) in place on a supporting member (20) such as a bed or table or on the body of a person while undergoing medical treatment. The terms equipment or assemblages include needles, gravity flow needles, straight intravenous applicators or catheters, plunger-type syringes, fluid carrying tubes, limb splinter member or the like. In one form, the retainer (10) includes an elongated, flexible strip (11) having a paper base (15) carrying a thin layer of closed cell foam (16) with an exposed covering of a cohesive coating (17). The opposite side of the base (15) carrying the foam (16) includes a mid-section of adhesive (18) covered by a removable tab (21) for detachably connecting the retainer (10) to a body limb or portion of the person or to a supporting structure (20).

- 1 -

RETAINER FOR MEDICAL DEVICES

BACKGROUND OF THE INVENTION

1.   Field of the Invention

The  present invention relates to medical appliance holders or support devices and, more particularly, to a novel disposable holder or retainer for detachably retaining a medical device or appliance in position about the limb or body part of a patient undergoing a medical procedure or onto a supporting member such as a bed, stand or table.

2.   Brief Description of the Prior Art

In the past, it has been conventional practice, by way of example, to insert the needle of a syringe,  catheter or the like into a vein of a patient undergoing medical treatment and the syringe as well as its attendant fluid feeding tubes are retained on the limb by adhesive tape which is wrapped about the syringe, tubing and the limb of the patient.  During normal medical procedure, it is routine practice to check fluid flow through the syringe by replacing the tubing periodically since collapse of the tubing is sometimes encountered when the fluid introduced to the patient via the syringe has been exhausted. During such a checking procedure, the tubing is removed from the installed syringe which sometimes causes the syringe needle to be inadvertently withdrawn from its insertion into the vein of the patient. Obviously, such a procedure is cumbersome and awkward as well as painful and inconvenient to the patient, especially when re-insertion of the syringe needle is required.

Furthermore, attempts have been made to provide special holding devices which are conformal to the shape and configuration of the syringe of appliance. The resultant devices are costly and do not fit a variety of shapes so that usage is limited to a few specific applications.

Therefore, a need has existed to provide a simple and economical means for retaining or holding a medical appliance in position during a medical procedure which may be readily attached to or detached from the patient or supporting structure and that may be disposed of after use. Such a holder must be easy to place upon the limb of the patient or a support and must be adapted to hold not only the medical appliance of specific shape but the tubes feeding fluid to the appliance as well regardless of shape or configuration.

## SUMMARY OF THE INVENTION

Accordingly, the above problems and difficulties are obviated by the present invention which provides a novel disposable retainer or holder for retaining conventional medical appliances in place on a patient's body or other adjacent supporting means while a person is undergoing a medical treatment or procedure. In one form, the retainer includes an elongated, flexible strip having a paper base supporting a foam layer on one side exposing a cohesive coating for self attachment about the medical appliance and supporting an adhesive coating at a mid-section portion for attachment to a body or support means. A covering tab is removably carried on the mid-section adhesive portion until ready for use.

Other versions of the invention are provided which employ specially configurated strips for securement to a variety of instruments, supports or limbs. Stiffening boards useful as splints or the like are readily incorporated into several versions for medical use.

Therefore, it is among the primary objects of the present invention to provide a novel medical appliance for supporting and retaining a medical device, tubing or the like while the appliance is in operating position on the limb of a person undergoing medical treatment.

Another object of the present invention is to provide a novel medical appliance retainer which is relatively inexpensive to manufacture so that the item may be

0206558

- 4 -

considered a disposable unit along with its attendant syringe, needle, tubing or the like.

Still another object of the present invention is to provide a novel supporting holder for a medical needle apparatus which is inserted into the limb or body portion of a person during a medical treatment so that the needle need not be removed during inspection or checking procedures necessary to remove the tubing from the needle device.

Still a further object of the present invention is to provide a novel holder for a medical appliance which may be readily attached or detached to the arm or limb of a patient or other supporting means so that the appliance is held in place and wherein the assembled holder and appliance may be discarded as a unit after use.

Yet another object of the present invention resides in the provision of a flexible retainer that is conformal to the shape of a variety of medical appliances for supporting and holding the appliance.

## BRIEF DESCRIPTION OF THE DRAWINGS

The features of the present invention which are believed to be novel are set forth with particularity in the appended claims. The present invention, both as to its organization and manner of operation, together with further objects and advantages thereof, may best be understood by reference to the following description, taken in connection with the accompanying drawings in which:

Figure 1 is front perspective view of the novel medical appliance holder incorporating the present invention which is illustrated in a typical medical procedure for retaining tubing in place;

Figure 2 is transverse cross-sectional view of the novel medical appliance retainer holder shown in Figure 1 as taken in the direction of arrows 2-2 thereof;

Figure 3 is a perspective view of the novel retainer shown in Figures 1 and 2 in a position preparatory for attachment to a supporting member or means;

Figure 4 is a perspective view of another embodiment of the present invention incorporating a rigid support or splint;

Figure 5 is a perspective view of still another embodiment of the present invention having portions for securement about the limb of a patient as well as other portions adapted for securement about a medical appliance;

Figure 6 is an end elevational view taken in the direction of arrows 6-6 of Figure 5; and

Figure 7 is a perspective view of still another version of the present invention employing a thickened mid-section of an elongated strip.

## DESCRIPTION OF THE PREFERRED EMBODIMENT

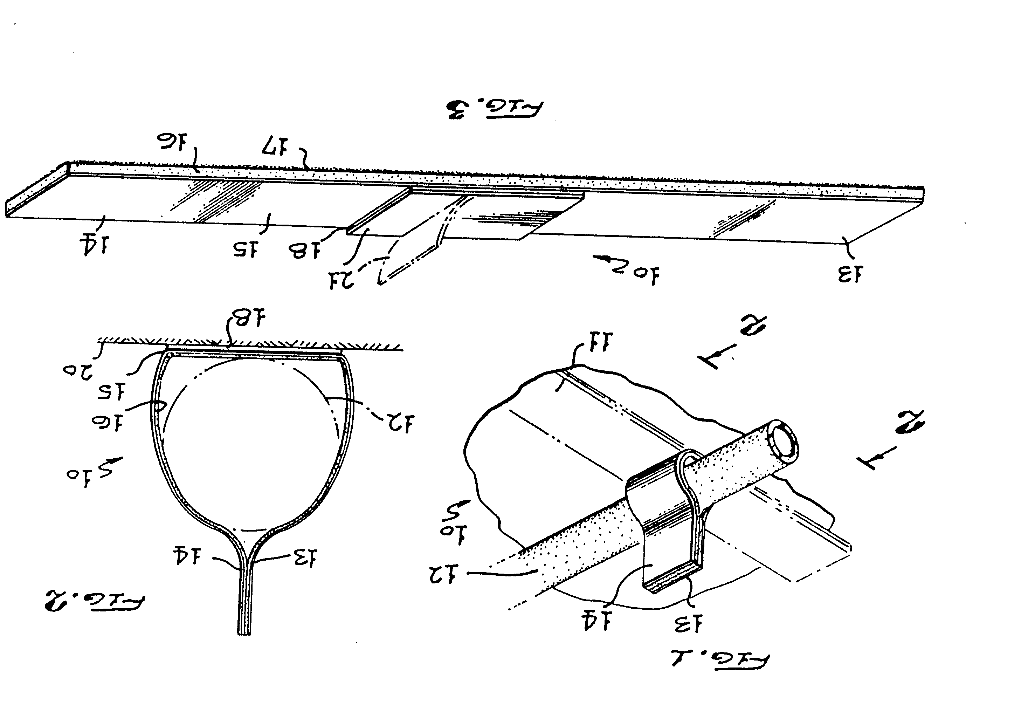

Referring to Figure 1, the novel medical appliance holder or retainer is illustrated in the general direction of arrow 10 which is illustrated in broken lines as an elongated strip 11 adapted to receive a medical appliance such as tubing 12 against one surface thereof so that the opposite ends 13 and 14 of the strip 11 may be folded about the appliance and joined together. Once the opposite ends are joined, the retainer 10 conforms to the shape of the appliance and firmly secures the appliance thereto by frictional fit.

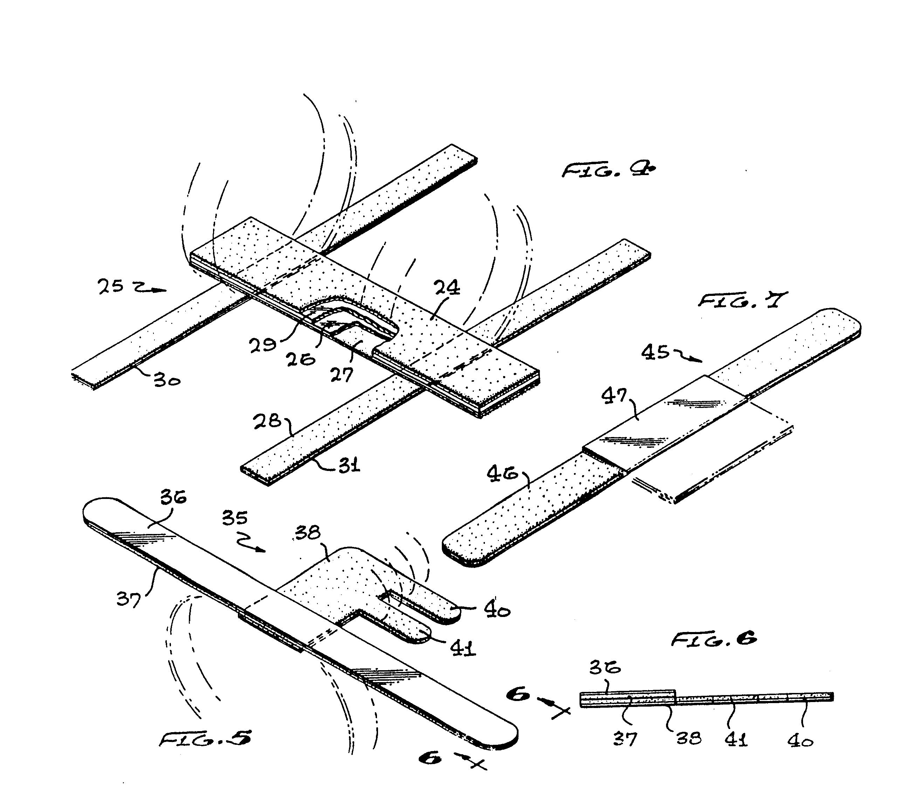

As shown more clearly in Figure 2, the holder or retainer 10 includes an elongated paper strip serving as a base 15 having one side adhered to a closed cell foam 16 which in turn is coated with an adhesive 17. Preferably, the foam is a closed cell polyurethane composition laminated to Kraft paper 15 on one side and the opposite side of the foam is coated with a cohesive coating adapted to adhere to itself when pressed together employing ordinary finger pressure. The opposite side of the Kraft paper from its side carrying the foam 17 is provided with a mid-section portion carrying an adhesive coating 18 readily attachable to a supporting surface such as a body portion of the patient, a bed sheet or nearby stand or table. The supporting surface is represented by numeral 20.

Referring now in detail to Figure 3, the retainer 10 is illustrated in its elongated form with the mid-section adhesive 18 covered by a releasable tab 21. The tab is illustrated as being partially removed in broken lines in order to expose the adhesive 18 therebeneath. Figure 3 further illustrates the foam 16 and the cohesive coating 17 carried thereon.

Therefore, in actual operation, it can be seen that the tab 21 may be readily removed to expose the adhesive 18 whereby the strip 11 may be placed on a supporting surface 20 so that the adhesive 18 will secure the strip thereto. Inasmuch as the adhesive 18 resides on the central mid-section of the strip, the opposite ends 13 and 14 be readily folded upon itself so as to encircle the medical appliance 12 in conformal relationship therewith until the opposing cohesive coating 17 on the respective ends are pressed together in securement. The securement is non-releasable and in order to remove the device 10 from the appliance, it is necessary to physically tear or rip the strip along any portion thereof not secured to

0206558

itself by the cohesive coating.

It is to be kept in mind that the inventive retainer may be employed for holding a variety of articles in position with respect to a supporting structure. For example, in place of the appliance 12, a bundle of wires may be secured to a supporting structure as well as conduit, pipes or other structural members requiring temporary or permanent securement.

Referring now in detail to Figure 4, another version of the present invention is illustrated taking the form of a disposable arm board 25 which may serve as a splint or in any other applications requiring a rigid support. The hold-down or retainer 25 includes an elongated stiffener or rigid board 26 carrying a cushion member 24 on one side thereof via an adhesive layer 29. The other side of the board or stiffener 26 carries a foam and cohesive layer as previously described and as indicated by numeral 27. The cohesive coating carried on foam 27 is exposed so as to be in contact with cohesive coating 28 carried on a similar foam layers and strips as previously described. In the present embodiment, a pair of strips are employed represented by numerals 30 and 31 which are arranged normal to the central longitudinal axis of the rigid board 26. Again, the cohesive coatings appearing on the strips 30 and 31 at their mid-sections come into engagement with the cohesive coating on the underside of the foam 27 carried on one side of the board 26. When in use, a broken limb may be placed against the cushion 24 while the opposite ends of the respective strips 30 and 31 are brought over around the board and the limb for pressure attachment as previously described with respect to the version shown in Figure 1. Once contact has been made between the cohesive coatings against the opposing surfaces of the opposite ends of each strip, securement takes place and the board 26 is firmly held against the limb. Diameter size of the limb is automatically compensated for when the opposing cohesive surfaces are pressed.

Referring now in detail to Figure 5, another embodiment of the invention is illustrated which may be referred to as a urinary catheter holder 35 which includes an elongated strip 36 which is identical to the previously described strips as indicated by numeral 11 and numeral 27 respectively. The strip 36 includes backing paper which is covered on one side by a foam 37 having an exposed cohesive coating adapted to readily engage and secure with an opposing cohesive coating 38 carried on a catheter holder represented by elongated portions 40 and 41. The elongated strip 36 may be placed about the leg of the user and the opposite ends of the strip join together on the underside of the leg so that the opposing surfaces of the cohesive layer attaches and secures. The catheter itself is placed against the surface of the coating 38 while the portions 40 and 41 are folded over so that the opposite ends of the portions engage with the edge marginal region of the coating 38 on the opposite side of the catheter disposed against the coating 38.

Referring now in detail to Figure 7, an endotracheal tube holder is illustrated in the general direction of arrow 45 which includes an elongated strip 46 as previously described with respect to strips 11, 31 and 36. However, the mid-section of the strip in reinforced with a chin portion indicated by numeral 47. A medical appliance and associated tubing may be held in place about the neck of a user when the chin is placed against the portion 47 while the opposite ends of the strip 46 are brought together so that the cohesive coating thereon engages and secures.

While particular embodiments of the present invention have been shown and described, it will be obvious to those skilled in the art that changes and modifications may be made without departing from this invention in its broader aspects and, therefore, the aim in the appended claims is to cover all such changes and modifications as fall within the true spirit and scope of this invention.

0206558

- 11 -

<u>CLAIMS</u>:

1. A retainer for supporting an article on a supporting surface comprising the combination of:

an elongated, flexible strip having a central longitudinal axis extending between opposite ends;

said strip includes a base sheet having opposite surfaces;

one surface of said sheet carries a closed cell foam composition layer over its entire surface area;

a cohesive coating carried on said foam composition layer and exposed for subsequent mated contact adhesion; and

the other of said sheet opposite surfaces having an adhesive coating carried on the mid portion thereof between said opposite ends for securement with the supporting surface.

2. The invention as defined in Claim 1 wherein: said strip opposite ends adapted to be folded towards each other about said mid porition to encircle the article so as to place said cohesive coating on said opposite ends in opposition with respect to each other for engagement to effect securement therebetween.

3. The invention as defined in Claim 2 including:

a cover tab removably attached to said adhesive coating.

4. The invention as defined in Claim 3 including:

a stiffening member on said strip to rigidize said strip;

cross straps carried on said strip in spaced apart relationship; and

each cross strap having opposite ends coated with a cohesive material for securement of said ends.

5. The invention as defined in Claim 1 including:

a base support having a cohesive coating of said strip so that said base support laterally extends outwardly therefrom; and

said base support having elongated portion cantilevered outwardly from a central base portion adapted to be folded over upon themselves for securement about the article via said cohesive coating.

6. A retainer for supporting an article on a supporting surface comprising the combination of:

an elongated, flexible strip conformal to the article having a central longitudinal axis extending between opposite ends;

said strip includes a base paper sheet having opposite surfaces;

one surface of said paper sheet carries a closed cell microfoam composition layer over its entire surface area;

- 13 -

a cohesive coating carried on said foam composition layer and exposed for subsequent mated contact adhesion;

the other of said sheet opposite surfaces having an adhesive coating carried on the mid-portion thereof between said opposite ends for securement with the supporting surface;

said strip opposite ends adapted to be folded towards each other about said mid-portion to encircle the article so as to place said cohesive coating on said opposite ends in opposition with respect to each other for bonded engagement to effect fixed securement therebetween;

a cover tab removably attached to said adhesive coating;

a stiffening member on said strip to rigidize said strip;

cross straps carried on said strip in spaced apart relationship;

each cross strap having opposite ends coated with a cohesive material for securement of said ends.

FIG.1

13    14

12

10
5

11

T
2

T
2

FIG.2

13    14

12    10
16
15
20
18

13    102    7    21

18    15    14

17    16

FIG.3

1/2

0206558

FIG. 4

FIG. 7

FIG. 5

FIG. 6

2/2

0206558

| | **DOCUMENTS CONSIDERED TO BE RELEVANT** | | EP 86304109.1 |
|---|---|---|---|
| Category | Citation of document with indication, where appropriate, of relevant passages | Relevant to claim | CLASSIFICATION OF THE APPLICATION (Int. Cl.4) |
| A | US - A - 3 826 254 (E.K.MELLOR)<br>* Totality *<br>-- | 1,6 | A 61 M 25/02<br>A 61 M 5/32 |
| A | US - A - 4 490 141 (M.A.LACKO et al.)<br>* Totality *<br>-- | | |
| A | US - A - 4 460 356 (D. MOSELEY)<br>* Totality *<br>-- | | |
| A | US - A - 4 333 468 (R.W.GEIST)<br>* Totality *<br>-- | | |
| A | CH - A - 546 068 (M. DECOURSEY)<br>* Totality *<br>-- | | **TECHNICAL FIELDS SEARCHED (Int. Cl.4)** |
| A | US - A - 4 457 754 (P. BUTTARAVOLI)<br>* Totality *<br>-- | | A 61 M 5/00<br>A 61 M 25/00 |
| A | US - A - 4 122 857 (R. HAERR)<br>* Totality *<br>-- | | |
| A | US - A - 4 336 806 (J. ELDRIDGE JR.)<br>* Totality *<br>---- | | |

The present search report has been drawn up for all claims

| Place of search | Date of completion of the search | Examiner |
|---|---|---|
| VIENNA | 25-09-1986 | LUDWIG |